# EUROPEAN PATENT SPECIFICATION

(11) **EP 0 622 069 B1**
(45) Date of publication and mention of the grant of the patent: **05.11.1997**
(21) Application number: 93600018.1
(22) Date of filing: 29.12.1993
(51) Int. Cl.: A61K 7/155

(54) **Enzymic preparations containing papain or chymotrypsin and methods of permanent enzymic depilation**
Enzymatische Zubereitungen die Papain oder Chymotrypsin enthalten zur dauerhafte enzymatische Entfernung von Haaren
Compositions enzymatiques contenant de la papaine ou de la chymotrypsine et procédé pour la dépilation enzymatique permanente

(30) Priority: 29.12.1992 GR 92010581
(43) Date of publication of application: 02.11.1994
(73) Proprietor: Protopapa, Evangelia, 145 65 Attikis (GR); Sekeris, Constantine E., GR-113 69 Athens, Kipseli (GR)
(72) Inventor: Protopapa, Evangelia, 145 65 Attikis (GR); Sekeris, Constantine E., GR-113 69 Athens, Kipseli (GR)

(56) References cited:
- FR-A- 2 259 906
- PARF. COSM. SAV. FRANCE vol. 1, no. 2 , 1971 , FRANCE pages 84 - 86 J.L. ABEGG 'les enzymes dans les préparations destinées aux soins de la chevelure et à l'épilation'
- PARFUMS COSMéTIQUES ARôMES no. 45 , 1982 , PARIS (FRANCE) page 43 F. JAPICA 'experimental determination of the effectiviness of the cosmetics which reduce the hairs' growth'
- CUOIO, PELLI, MATER. CONCIANTI vol. 44, no. 4 , 1968 , NAPOLI (ITALY) pages 347 - 356 A. SIMONCINI 'unhairing activity of proteolytic enzymes'

## Description

The present invention refers to depilatory preparations containing the proteolytic enzymes papain or chymotrypsin, with the aim of application, with the suitable method of ionization, for permanent enzymic depilation for all types of skin, ranging from dry, sensitive, to fatty, resistant ones.

The up to now in use technic of permanent enzymic depilation is based on preparations containing papain, in concentrations not clearly defined, in the presence of glutathione, EDTA and propylene glycol, as stabilizers of enzymic activity. The not clearly defined concentration of the enzyme papain does not permit the differential application, depending on the peculiarities of each type of skin. No analogous research exists for the use of enzymic methods for depilation, with the exception of older work of Australian researchers on removal of the wool of sheep. The present invention introduces as depilatory preparation the enzyme papain, in concentrations selected for different skin types, in contrast to the up to now used preparations, with undefined concentrations of the enzyme. The present invention introduces also the use for the first time, as depilatory agent, the enzyme chymotrypsin, in concentrations selected for different skin types. The present invention introduces the use of dithiothreitol and ethylene glycol as stabilizers of enzyme activity, leading to improvement in comparison to the up to now in use stabilizers. In addition, the present invention refers to a method of depilation, which applies the preparations of the enzyme papain or the enzyme chymotrypsin for the depilation of any type of skin, in concentrations of 0,1mg/ml to 1mg/ml, as well as to the method of depilation which is based on the sequential application of the preparation papain, followed by that of the respective preparation which contains chymotrypsin, in concentrations of 0,1mg-1mg/ml, depending on the peculiarities of each skin type. The application of depilatory preparations which contain either papain, or chymotrypsin, or the sequential application first of the preparation containing papain, followed by the preparation containing chymotrypsin, is performed by the method of ionophoresis for suitable time periods and with suitable current intensities. Based on our own measurements the time period of application of ionophoresis is 1.5 to 2.5 min for each enzyme preparation and the intensity of current is 1,5-2,5 mAmp, independent of the skin type. Our own observations showed that the depilatory action is correlated to the concentration of the enzyme in the preparations and not to the time period of ionophoresis, if this superpasses 1,5 min.

As our own published experimental studies have shown for the first time in the international bibliography, the action of papain leads to damage of the reproductive cells of the hair follicle, which explains the positive results in depilation. In these studies, we observed that the enzyme chymotrypsin also leads to damage of the reproductive cells and of the hair follicle, in fact with more intense action, in comparison to the respective concentrations of preparations containing papain. Our invention improves, due to application by ionophoresis of preparations with known concentrations of the enzyme papain, due to the application with ionophoresis for the first time of preparations with known concentrations of the enzyme chymotrypsin, due to the introduction of sequential application with ionophoresis first of preparations of papain, followed by that of preparations of chymotrypsin, due to the presence in the preparations as stabilizers of enzyme activity of dithiothreitol and ethylene glycol, and the thus offered possiblity for the depilation of different types of skin, from the dry-sensitive to the fatty-resistant, the up to now in use method of enzymic depilation. We also observed that the sequential application first of papain, followed by chymotrypsin, leads to intense damage of the hair follicle and that the application first of papain and then of chymotrypsin gives clearly better results, than the use of only one of the depilatory preparations, for all concentrations used. We also ascertained that the consecutive application first a preparation containing papain, followed by the application of the same papain preparation, is inferior in activity, from that of the sequential application of a papain preparation followed by the application of a chymotrypsin preparation. Furthermore we ascertained that the sequential application of papain and chymotrypsin, even in concentrations of O,1mg/ml, had also an important result on the hair follicle, a fact which renders these preparations with the low enzyme concentrations, suitable for application on dry, sensitive, regions of skin. We also observed that the use of high concentrations of enzymes on dry, sensitive skins, leads to intense depilatory action but provokes also intense irritation. For this reason in these cases the application of low enzyme concentrations is advisable. Therefore, the production of preparations which contain papain and chymotrypsin in two different concentrations and the sequential application of papain and chymotrypsin offers an important improvement in the methodology of enzymic depilation. In addition, the use of preparations with intermediate enzyme concentrations for intermediate skin types offers important improvement in the methodology of enzymic depilation.

The proteolytic enzymes papain and chymotrypsin in absolutely pure state will be used. The enzymes will be stored in the presence of lactose or other dilutant in powder form, keeping thus the enzymes in stabile, undenatured, state, whereas the enzyme stabilizers, dithiothreitol, ethylendiaminotetraacetic (EDTA) and ethylene glycol, will be stored in a liquid buffered solution, of suitable pH for each enzyme. Thus the buffer solution which is intendent for the enzyme papain will have a pH of 5,0-6,0, whereas the respective for chymotrypsin pH will be 7,2-8,0, due to the fact that the optimun pH for the enzymes papain and chymotrypsin lies towards the acidic (pH 5-5,5) and slightly alkaline (pH 7,8), respectively. As buffer solution a mixture of sodium hydrogen phasphate and sodium phosphate for pH 5,0-6,0 and tris(hydroxymethyl) amino-methane-hydrochloric acid for pH 7,2-8,0 was chosen, although other buffer solutions, which cover the desired pH range can also be used.

Both, the enzymes with the adjuvans, and the liquid part, should be kept at 2-6°C so that the enzyme will retain intact its activity and in the liquid part the dethiothreitol remain in active form, because at room temperature it loses progressively its activity.

Immediately before use, the enzyme will be dissolved in the liquid part, with the formation of a solution, which, stored at 2-6°C retains its depilatory activity at least for 7 days and if stored at temperatures of (15°)-(20°) at least for 4 weeks.

According to one proposed realization of the invention, from 0,1mg up to 1mg of the enzyme papain or of the enzyme chymotrypsin, will be placed in flasks together with 30-70mg lactose, 60-80mes, as dilutant. In 1ml ampules will be placed 0,5-1,5mg dithiothreitol, 0,015-0,35mg ethylenediaminotetraacetic acid (EDTA), 0,10-0,30ml ethylene glycol and 0,70-0,90ml buffer solution, pH 7,2-8,0, if it refers to solution in which papain will be dissolved and pH 5-6,0, if it refers to solution in which chymotrypsin will be dissolved. Both, the flasks containing the enzyme and the ampules containing the stabilizing substances of the enzymic activity, will be stored at temperatures 2-6°C. Before use, the content of the flasks will be dissolved by adding the 1ml solution of the ampules. The presence of dithiothreitol, which protects the thiol groups of the enzymes, of EDTA and of ethylene glycol (in an end concentration of 10-30%), act by stabilizing the enzymes, so that after their dissolution they will be kept in an active state at least for one week, if the solution is kept at temperatures of 2-6°C.

In this way we offer lastly for use:
a) Solutions of papain from 0.1 up to 1mg/ml at pH 5,0-6,0, according to a proposed realizatin of the invention pH 5,9.
b) Solutions of chymotrypsin from 0,1 up to 1mg/ml at pH 7,2-8,0, according to a proposed realization of the invention pH 7.4.

For regions of fatty, resistant skins the solutions of high concentrations of enzymes will be used, whereas in regions of dry, sensitive skins the low enzyme concnetrations will be used. It is up to the judgement of the specialist to sequentially use solutions of high concentrations of one enzyme and low of the other, or vice versa, of low concentrations of the first enzyme and high of the second, as well as preparations of intermediate enzyme concentrations.

The application of the enzyme preparations will be made with the method of ionophoresis, the time period of application will be 1,5 up to 2,5 min for every enzyme preparation and the current intensity 1,5-2,5 mAmp, independent of skin type.

## Claims

1. Depilatory preparation in liquid form, which is based on the action of the enzyme papain, applied by ionophoresis, characterized by the fact that the concentrations of enzyme are from 0,1mg/ml to 1mg/ml, which also contains as enzyme stabilizers dithiotreitol, in concentrations of O,5-1,5mg/ml, EDTA, in concentrations of 0,015-0,035mg/ml, ethylene glycol, in concentrations of 0,1-0,3ml/ml as well as lactose as adjuvans, in concentration of 30-70mg/ml, in buffer solution sodium hydrogen phosphate - sodium phosphate, pH 5,0-6,0, which covers the region of pH 5,0-5,5, which is the pH optimum of action of the enzyme papain.

2. Depilatory preparation according to claim 1, characterized by the fact that the concentration of the enzyme papain is 0,1mg/ml which renders the depilatory preparation suitable for dry and sensitive skins.

3. Depilatory preparation according to claim 1, characterized by te fact that the concentration of the enzyme papain is 1mg/ml, which renders the depilatory preparation suitable for fatty and resistant skins.

4. Depilatory preparation in liquid from, which is based on the action of the enzyme chymotrypsin, applied by ionophoresis, characterized by the fact that the concentrations of the enzyme are form 0,1mg/ml to 1mg/ml, which also contains as enzyme stabilizers EDTA, in concentrations of 0,015-0,35 mg/ml, ethylene glycol, in concentrations of 0,1-0,3ml/ml as well as lactose, as adjuvans, in concentrations of 30-70 mg/ml, in buffer solution tris-hydroxymethylamino-methane-hydrochloric acid pH 7,2-8,0, which covers the region of pH 7,8, which is the pH optimum of action of theenzyme chymotrypsin.

5. Depilatory preparation according to claim 4 characterized by the fact that the concentration of the enzyme chymotrypsin is 0,1mg/ml, which renders the depilatory preparation suitable for dry and sensitive skins.

6. Depilatory preparation according to claim 4, characterized by the fact that the concentration of the enzyme chymotrypsin is 1mg/ml which renders the depilatory preparation suitable for fatty and resistat skins.

7. Method of depilation which uses the depilatory preparations according to claim 1 or 2 or 3 or 4 or 5 or 6, characterized by the fact that the application on the skin is made by ionophoresis, whereby the time period of application of ionophoresis is from 1,5-2,5 min and the current intensity is from 1,5-2,5 mAmp.

8. Method of depilation which uses first one preparation according to claims 1 or 2 or 3, followed by the sequential application of the preparation according to claims 4 or 5 or 6, characterized by the fact that the application on the skin is made by ionophoresis, whereby the time period of application of ionophoresis is from 1,5-2,5 min and the current intensity is from 1,5-2,5 mAmp, for each preparation.

9. Method of depilation according to claim 8, characterized by the fact the concentration of enzymes contained in the preparations are 0,1mg/ml, suitable for dry and sensitive skins.

10. Methods of depilation according to claim 8, characterized by the fact that the concentrations of enzymes contained in the preparations are 1mg/ml, suitable for fatty and dry skins.

11. Method of depilation according to claim 8 characterized by the fact that the concentrations of the enzymes which are contained in the preparations are intermediates of concentrations 0,1 and 1mg/ml, suitable respectively for intermediate particularities of skin types.

## Patentansprüche

1. Enthaarungspräparat in flüssiger Form, welches auf der Wirkung der Enzyme Papain basiert, angewendet wird mittels Ionophoresis, gekennzeichnet durch die Tatsache daß die Konzentrationen der Enzyme von 0,1 mg/ml bis 1 mg/ml reichen, welches zudem als Enzym-Stabilisatoren Dithiotreitol in Konzentrationen von 0,5 - 1,5 mg/ml, EDTA in Konzentrationen von 0,015 - 0,035 mg/ml, Ethylenglykol in Konzentrationen von 0,1 - 0,3 ml/ml, ebenso wie Lactose als unterstützendes Mittel in Konzentrationen von 30--70mg/ml enthält, in einer Pufferlösung Natriumwasserstoffphosphat - Natriumphosphat, pH Wert 5,0 - 6,0, welcher den pH - Bereich von 5,0 - 5,5 abdeckt, welches der optimale pH-Wert für die Wirkung des Enzyms Papain ist.

2. Enthaarungspräparat gemäß Anspruch 1, gekennzeichnet durch die Tatsache, daß die Konzentration des Enzyms Papain 0,1 mg//ml beträgt, was das Enthaarungspräparat für trockene und sensible Haut geeignet macht.

3. Enthaarungspräparat gemäß Anspruch 1, gekennzeichnet durch die Tatsache, daß die Konzentration des Enzyms Papain 1 mg/ml beträgt, was das Enthaarungspräparat für fettige und widerstandsfähige Haut geeignet macht.

4. Enthaarungspräparat in flüssiger Form, welches auf der Wirkung des Enzyms Chymotrypsin basiert, angewendet wird mittels Ionophoresis, gekennzeichnet durch die Tatsache, daß die Konzentrationen des Enzyms von 0,1 mg/ml bis 1 mg/ml reichen, wlches außerdem als Enzymstabilisatoren EDTA in Konzentrationen von 0,015 - 0,35 mg/ml, Ethylenglykol in Konzentrationen von 0,1 - 0,3 ml/ml ebenso wie Lactose als uunterstützendes Hilfsmittel in Konzentrationen von 30--70 mg/ml enthält, in Pufferlösung Tris - hydroxmethylamino - methan - Salzsäure, pH-Wert 7,2 - 8,0, welche den Bereich des pH--Wertes 7,8 abdeckt, welcher das pH Optimum für die Wirkung des Enzyms Chymatrypsin ist.

5. Enthaarungspräparat gemäß Anspruch 4, gekennzeichnet durch die Tatsache, daß die Konzentration des Enzyms Chymotrypsin 0,1 mg/ml beträgt, was es als Enthaarungspräparat für trockene und sensible Haut geeignet macht.

6. Enthaarungspräparat gemäß Anspruch 4, gekennzeichnet durch die Tatsache, daß die Konzentration des Enzyms Chymotrypsin 1 mg/ml beträgt, was das Enthaarungspräparat für fettige und widerstandsfähige Haut geeignet macht.

7. Methode der Enthaarung, welche für die Enthaarungspräparate gemäß den Ansprüchen 1 oder 2 oder 3 oder 4 oder 5 oder 6 verwendet wird, gekennzeichnet durch die Tatsache, daß die Anwendung auf der Haut mittels Ionophoresis geschieht, wodurch der Zeitraum der Anwendung der Ionophoresis zwischen 1,5 - 2,5 min reicht, und die Stromstärke von 1,5 - 2,5 mAmp.

8. Methode der Enthaarung, bei welcher zuerst ein Präparat gemäß Anspruch 1 oder 2 oder 3 verwendet wird, gefolgt von der aufeinanderfolgenden Anwendung des Präparats gemäß den Ansprüchen 4 oder 5 oder 6, gekennzeichnet durch die Tatsache, daß die Anwendung auf der Haut mittels Ionophoresis geschieht, wodurch die Zeitspanne der Anwendung der Ionophoresis von 1,5 - 2,5 min reicht, und die Stromstärke von 1,5 - 2,5 mAmp, für jedes Präparat.

9. Methode der Enthaarung gemäß Anspruch 8, gekennzeichnet durch die Tatsache, daß die Konzentration der enthaltenen Enzyme 0,1 mg/ml beträgt, geeignet für trockene und sensible Haut.

10. Methode der Enthaarung gemäß Anspruch 8, gekennzeichnet durch die Tatsache, daß die Konzentration der in den Präparaten enthaltenen Enzyme 1 mg/ml beträgt, geeignet für fettige und trockene Haut.

11. Methode der Enthaarung gemäß Anspruch 8, gekennzeichnet durch die Tatsache, daß die Konzentration der Enzyme, die in den Präparaten enthalten sind, in der Mitte liegen zwischen 0,1 und 1 mg/ml, jeweils geeignet für dazwischenliegende Eigenheiten von Hauttypen.

## Revendications

1. Préparation épilatoire sous forme liquide, qui est basée sur l'action de l'enzyme papaine, appliquée par ionophorèse, caractérisée du fait que les concentrations des enzymes sont 0,1 mg/ml - 1 mg/ml, contenant aussi en tant que stabilisateurs enzymatiques threitol disulphide, dont la concentration est 0,5 - 1,5 mg/ml, EDTA, dont la concentration est 0,015 - 0,35 mg/ml, glycol éthylénique, dont la concentration est 0,1 - 0,3 mg/ml, ainsi que lactose comme supplémentaire, dont la concentration est 30 - 70 mg/ml, dans de solutions régulatrices de sodium hydrophosphorique et de sodium phosphorique pH 5,0 - 6,0, qui couvre la region pH 5,0 - 5,5, qui constitue le meilleur pH pour l'action de l'enzyme papaine.

2. Préparation épilatoire conformément à la prétention 1, caractérisée du fait que la concentration de l'enzyme papaine est 0,1 mg/ml, ce qui rend la préparation épilatoire propre aux peaux sèches et délicates.

3. Préparation épilatoire, conformément à la prétention 1, caractérisée du fait que la concentration de l'enzyme papaine est 1 mg/ml, ce qui rend la préparation épilatoire propre aux peaux grasses et résistantes.

4. Préparation épilatoire sous forme liquide, basée sur l'action de l'enzyme chymotrypsine, appliquée par ionophorèse, caractérisée du fait que les concentrations des enzymes sont 0,1 mg/ml - 1 mg/ml, contenant aussi comme stabilisateurs enzymatiques EDTA, dont la concentration est 0,015 - 0,35 mg/ml, glycol éthylénique, dont la concentration est 0,1 - 0,3 mg/ml ainsi que lactose comme supplémentaire, dont la concentration est 30 - 70 mg/ml dans de solutions régulatrices d'acide tri(hydroxyméthyl)aminométhanochlorique, pH 7,2 - 8,0, couvrant la région pH 7,8, qui constitue le meilleur pH pour l'action de l'enzyme chymotrypsine.

5. Préparation épilatoire conformément à la prétention 4, caractérisée du fait que la concentration de l'enzyme chymotrypsine est 0,1 mg/ml, ce qui rend la préparation épilatoire propre aux peaux sèches et délicates.

6. Préparation épilatoire conformément à la prétention 4, caractérisée du fait que la concentration de l'enzyme chymotrypsine est 1 mg/ml, ce qui rend la préparation épilatoire propre aux peaux grasses et résistantes.

7. Méthode d'épilation, qui utilise les préparations épilatoires conformément à la prétention 1 ou 2 ou 3 ou 4 ou 5 ou 6, caractérisée du fait que l'application à la peau est réalisée par ionophorèse, tandis que la durée de l'application de l'ionophorèse varie de 1,5 à 2,5 min. et le voltage du courant est 1,5 - 2,5 mAmp.

8. Méthode d'épilation, qui utilise premièrement une préparation conformément aux prétentions 1 ou 2 ou 3 et après on procède à l'application successive de la préparation conformément aux prétentions 4 ou 5 ou 6, caractérisée du fait que l'application à la peau est réalisée par ionophorèse, tandis que la durée de l'application de l'ionophorèse varie de 1,5 à 2,5 min. et le voltage de 1,5 à 2,5 mAmp. pour chaque préparation.

9. Méthode d'épilation conformément à la prétention 8, caractérisée du fait que la concentration des enzymes, qui sont contenus dans les préparations, est 0,1 mg/ml propre aux peaux sèches et délicates.

10. Méthode d'épilation conformément à la prétention 8, caractérisée du fait que la concentration des enzymes, qui sont contenus dans les préparations, est 1 mg/ml, propre aux peaux sèches et délicates.

11. Méthode d'épilation conformément à la prétention 8, caractérisée du fait que la concentration des enzymes, qui sont contenus dans les préparations, est intermédiaire de concentrations 0,1 et 1 mg/ml, propre respectivement aux particularités intermédiaires de types de la peau.
